# EUROPEAN PATENT SPECIFICATION

(11) **EP 2 429 563 B1**
(45) Date of publication and mention of the grant of the patent: **09.07.2014**
(21) Application number: 10736805.2
(22) Date of filing: 06.05.2010
(51) Int. Cl.: A61K 38/08, A61K 38/10, A61P 35/00, A61K 38/17

(54) **PEPTIDE ABLE TO DISRUPT THE PROTEIN COMPLEX BETWEEN THE HIS273 MUTATED P53 PROTEIN AND THE ONCOSUPPRESSIVE P73 PROTEIN IN TUMOUR CELLS AND THERAPEUTIC USES THEREOF**
PEPTIDE ZUR STÖRUNG DES PROTEIN-KOMPLEX ZWISCHEN DEM HIS273 MUTIERTEN P53-PROTEIN UND DEM ONCOSUPPRESSIVEN P73-PROTEIN IN TUMORZELLEN UND DEREN MEDIZINISCHE VERWENDUNG
PEPTIDE PERTURBANT LE COMPLEXE PROTÉIQUE ENTRE LA PROTÉINE P53 MUTÉE HIS273 ET LA PROTÉINE ONCOSUPPRESSIVE P73 DANS DES CELLULES TUMORALES ET SES UTILISATIONS THÉRAPEUTIQUES

(30) Priority: 11.05.2009 IT RM20090232
(43) Date of publication of application: 21.03.2012
(73) Proprietor: Istituti Fisioterapici Ospitalieri, 00128 Roma (IT)
(72) Inventor: CITRO, Gennaro, 00182 Roma (IT); BLANDINO, Giovanni, I-00128 Roma (IT); STRANO, Sabrina, I-00128 Roma (IT); DI AGOSTINO, Silvia, I-00128 Roma (IT)
(74) Representative: Gitto, Serena
(86) International application number: PCT/IT2010/000203
(87) International publication number: WO 2010/131283

(56) References cited:
- WO-A1-2006/054138
- WO-A2-03/025010
- DI AGOSTINO SILVIA ET AL: "The disruption of the protein complex mutantp53/p73 increases selectively the response of tumor cells to anticancer drugs." CELL CYCLE (GEORGETOWN, TEX.) 1 NOV 2008, vol. 7, no. 21, 1 November 2008 (2008-11-01), pages 3440-3447, XP002568712 ISSN: 1551-4005 cited in the application
- LAIN S: "If p53 can't do it, ask p73" CELL CYCLE 20081101 US, vol. 7, no. 21, 1 November 2008 (2008-11-01), pages 3290-3291, XP002568714
- IRWIN MEREDITH S ET AL: "Chemosensitivity linked to p73 function." CANCER CELL APR 2003, vol. 3, no. 4, April 2003 (2003-04), pages 403-410, XP002568713 ISSN: 1535-6108

### SEQUENCE LISTING

<110> Istituti Fisioterapici Ospitalieri
<120> Peptide able to disrupt mp53 HIS273/p73 proteic complexes in tumour cells and uses thereof in medical field
<130> PCT27880
<160> 16
<170> PatentIn version 3.5
<210> 1
   <211> 12
   <212> PRT
   <213> artificial
<220>
   <223> p73 SIMP (aa 131-142)
<400> 1
   Phe Gln Gln Ser Ser Thr Ala Lys Ser Ala Thr Trp
   1               5                   10
<210> 2
   <211> 10
   <212> PRT
   <213> artificial
<220>
   <223> p73 SIMP (aa 146-155)
<400> 2
   Pro Leu Leu Lys Lys Leu Tyr Cys Gln Ile
   1               5                   10
<210> 3
   <211> 12
   <212> PRT
   <213> artificial
<220>
   <223> p73 SIMP (aa 159-170)
<400> 3
   Cys Pro Ile Gln Ile Lys Val Ser Thr Pro Pro Pro
   1               5                   10
<210> 4
   <211> 12
   <212> PRT
   <213> artificial
<220>
   <223> p73 SIMP (aa 174-186)
<400> 4
   Ile Arg Ala Met Pro Val Tyr Lys Lys Ala Glu His
   1 5 10
<210> 5
   <211> 9
   <212> PRT
   <213> artificial
<220>
   <223> p73 SIMP (aa 190-198)
<400> 5
   Val Val Lys Arg Cys Pro Asn His Glu
   1 5
<210> 6
   <211> 13
   <212> PRT
   <213> artificial
<220>
   <223> p73 SIMP (aa 200-211) wherein Q has been inserted between G (aa200) and R (aa201)
<400> 6
   Gly Gln Arg Asp Phe Asn Glu Gly Gln Ser Ala Pro Ala
   1 5 10
<210> 7
   <211> 9
   <212> PRT
   <213> artificial
<220>
   <223> p73 SIMP (aa 215-223)
<400> 7
   Ile Arg Val Glu Gly Asn Asn Leu Ser
   1 5
<210> 8
   <211> 12
   <212> PRT
   <213> artificial
<220>
   <223> p73 SIMP (aa 227-238)
<400> 8
   Asp Asp Pro Val Thr Gly Arg Gln Ser Val Val Val
   1 5 10
<210> 9
   <211> 11
   <212> PRT
   <213> artificial
<220>
   <223> p73 SIMP (aa 244-253) wherein G has been inserted before Q (aa 244)
<400> 9
   Gly Gln Val Gly Thr Glu Phe Thr Thr Ile Leu
   1 5 10
<210> 10
   <211> 14
   <212> PRT
   <213> artificial
<220>
   <223> p73 SIMP (aa257-269) wherein N has been inserted between N(aa257) and C (aa258)
<400> 10
   Met Asn Cys Asn Ser Ser Cys Val Gly Gly Met Asn Arg Arg
   1 5 10
<210> 11
   <211> 11
   <212> PRT
   <213> artificial
<220>
   <223> p73 SIMP (aa273-283)
<400> 11
   Ile Ile Ile Thr Leu Glu Met Arg Asp Gly Gln
   1 5 10
<210> 12
   <211> 11
   <212> PRT
   <213> artificial
<220>
   <223> p73 SIMP (aa287-297)
<400> 12
   Arg Arg Ser Phe Glu Gly Arg Ile Cys Ala Cys
   1 5 10
<210> 13
   <211> 10
   <212> PRT
   <213> artificial
<220>
   <223> p73 SIMP (aa301-310)
<400> 13
   Asp Arg Lys Ala Asp Glu Asp His Tyr Arg
   1 5 10
<210> 14
   <211> 13
   <212> PRT
   <213> artificial
<220>
   <223> p73 SIMP (aa 190-202)
<400> 14
   Val Val Lys Arg Cys Pro Asn His Glu Leu Gly Arg Asp
   1 5 10
<210> 15
   <211> 12
   <212> PRT
   <213> artificial
<220>
   <223> SEQ ID NO:1 wherein K is substituted by A
<400> 15
   Phe Gln Gln Ser Ser Thr Ala Ala Ser Ala Thr Trp
   1 5 10
<210> 16
   <211> 12
   <212> PRT
   <213> artificial
<220>
   <223> SEQ ID NO:1 wherein k is substituted by R
<400> 16
   Phe Gln Gln Ser Ser Thr Ala Arg Ser Ala Thr Trp
   1               5                   10

## Description

The present invention concerns peptide able to disrupt the protein complex between HIS273 mutated p53 protein and oncosuppressive p73 protein in tumor cells and uses thereof in medical field. More particularly, the present invention concerns a SIMP peptide (Short-interfering mutant p53 peptides) suitable to disrupt the protein complexes within tumour cells resulting from m-p53 and p73 proteins selectively in tumours wherein m-p53 contains His273 mutation.

During the last years the research interest moved from conventional treatments with cytotoxic drugs towards therapies using a single intelligent drug or in combination with standard therapies. Since the positive therapeutic outcome is directly proportional to typization of patient subpopulations with a specific genomic or methabolomic profile, the research interest has been focused on studies aiming to understand the activity mechanism of cancer associated genes. Among the latter, p53 oncosuppressor gene products plays an important role.

It is known that p53 family members, including p53, p63, p73 proteins and relevant isoforms thereof, are involved in key processes relating to cellular viability as the inhibition of cell growth, apoptosis, senescence and differentiation. It is known (Strano et al., Journal Biological Chemistry 275:29503-508 2000; Strano et al., Journal Biological Chemistry 277: 18817-26.2002) the occurrence in tumour cell lines of protein complexes from p53/p73 mutated proteins. The formation of such complexes inhibits the p73 transcription and apoptotic activity as a response to antitumor drugs (Strange S. Blandino G. Cell Cycle 2:348 - 9 2003) weakening and inactivating the p73 antitumor activity. Therefore, m-p53/p73 complex exerts its oncogenic function preventing the p73 exact positioning on regulation regions of pro-apoptotic target genes, resulting in an increase of m-p53/p73 bond entrapped amount.

The analysis of said protein complexes evidenced that contact surfaces involved in m-p53/p73 bond are represented in both cases by DNA bonding domain (DBD). DBD represents the mutation site of the most mutations occurring in the human tumours. Moreover, m-p53 core (DNA multiprotein complexes including transcription factors, acetylases deacetylases, anchor proteins cooperating with m-p53 to activate or repress target genes.

m-p53 proteins are in large amount within tumour cells. Consequently, also only the partial reactivation of a small protein aliquot to m-p53 could be sufficient to restore the oncosuppressive functions thereof. acid sequence largely resembles the sequence of p73 DNA bonding domain (European Patent EP1812467).

p73 protein consists of 636 amino acids subdivided in 4 portions: i.e. N-terminal representing transcription activation domain (1 - 54); middle representing specific DNA bonding domain (131-310); oligomerization domain (345 - 390); and finally a C-terminal portion (390 - 636). In order to cover the entire middle region, representing specific DNA bonding domain, 14 peptides have been designed, 12 of which are described in EP1812467, SIMP5M is described in DiAgostino et al., Cell Cycle). SIMP peptide sequences drawn in DNA bonding domain of p73 human gene are reported in Table 1.

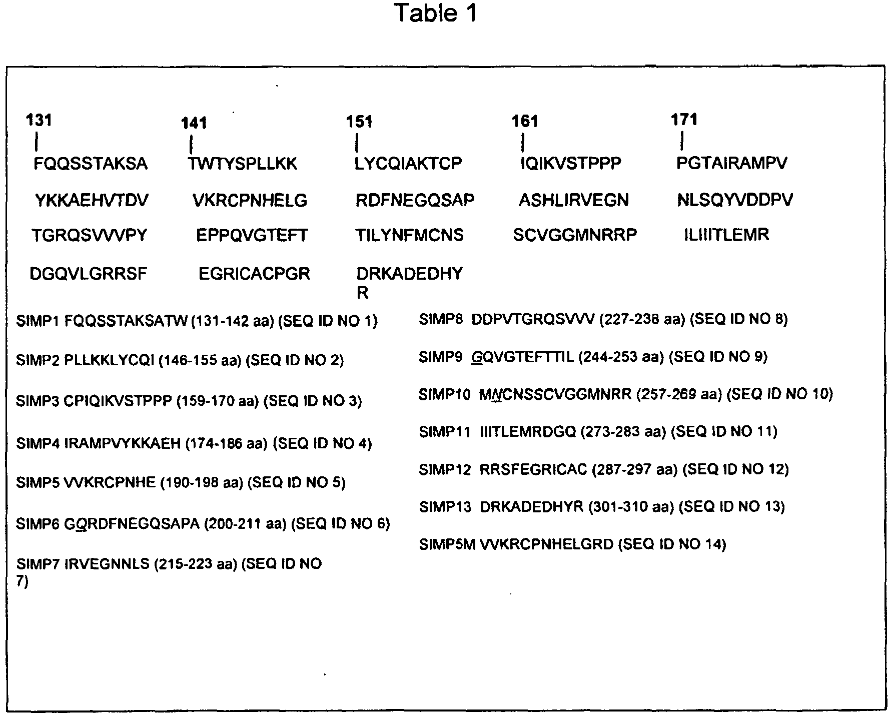

The length of peptides has been established according to their pharmacological application. In fact, SIMP peptides, having a length from 8 to 10 amino acids, are suitable to permeate the cytoplasmic and nuclear membrane. Above mentioned peptides are suitable to disrupt complexes occurring within the nucleus of m-p53/p73 tumour cells, particularly in the presence of a His- 175 or His-273 (p-53-His 175, p-53-His 273) mutation

In order to detect the minimal regions involved or engaged in p73 and m-p53 protei n in teraction, said p eptides have been subdivided in three subgroups and going on by removal, two peptides, i.e. SIMP5 and SIMP6, more suitable to restore the p73 oncosuppressive functions, have been characterized. At the end of the experiments, the peptides located within the middle domain of p73 sequence displayed to be suitable in disrupting the m-p53/p73 complex. SIMP5 and SIMP6 molecules carry out functions selectively aiming to the elimination of tumour cells containing specific mutations of p53 gene and it does not seem that said molecules affect cellular systems lacking in p53 gene or containing p53 wild gene. In fact, such molecules make SKBR3 tumour lines, containing his-175 mutation, more sensitive to chemotherapeutic treatment (Di Agostino et al., Cell Cycle 7:21,3440-3447. 2008). Analogous experiments carried out using breast carcinoma cell lines containing other types of mutation do not induce changes in the apoptotic response (for example: SW480 and MDA-MB-468). The restoration of the chemosensitivity is directly correlated to p53 mutation type.

These peculiarity and specificity make SIMP molecules suitable to a specific and personalized therapy and with pharmacological interest. However, above mentioned peptides up to now are known as being suitable to disrupt m-p53/p73 complexes in tumour cells containing His 175 mutations of p53 gene and not different mutations, therefore they are considered not suitable to restore the m-p53 oncosuppressive function in many tumours of same or different isotype but containing other mutation categories. In the light of above it is therefore apparent the need to provide new molecules suitable to disrupt m-p53/p73 complexes in tumour cells overcoming the disadvantages of known peptides.

The authors of the present invention now surprisingly found that SIMP1: FQQSSTAKSATW (SEQ ID NO: 1) peptide is suitable to disrupt m-p53/p73 complexes in tumour cells containing his-273 mutation, which is the most diffused mutation in various isotype human tumours. Particularly, the authors of the present invention now found that a mutated version of SIMP1 peptide, wherein K138 residue has been substituted with an Arginine residue, displays the same functions as SIMP1 peptide thus increasing activities thereof.

SIMP1 peptide: FQQSSTAKSATW (SEQ ID NO: 1) (MW: 1341, PI: 8.75; 131-142 aa of p73α human gene sequence) is located in the middle region of specific DNA bonding domain from 131 to 310 aa. By integrating the information from data obtained by the present authors with literature already known structural and crystallographic data, interfering peptides have been located on p53 DNA bonding surface (Kitayner et al., Mol. Cell.22; 741-53. 2006). A p73 model based on the structure of a p53 monomer has been constructed. For initial model Insightll (Accelrys, San Diego, CA) homology module and for minimization energy Discover3 module, using force field CVFF, have been used. The model of p73/p53 dimer has been obtained by p73 appropriate localization in a monomer within p53 symmetrical dimer p53 (Di Agostino et al., Cell Cycle 7:21,3440-3447. 2008 ;Kitayner et al., Mol.Cell.22;741-53. 2006)..

It has been observed that SIMP1 disrupts mutp53/p73 interaction by interfering near the DNA p53 binding surface already weakened as result of His-273 mutation occurrence, while SIMP5 is interface mapped preventing the formation of mut-p53 His-175/p73 dimer. The SIMP1 DNA interaction is an electrostatic one.

Where SIMP1 is mutated to Alanina at K138 site, the complexes do not seem to be conformationally modified, while the contrary occurs when K138 residue is modified to R. In such a case, structure interfering effects increase.

SIMP1 KO: FQQSSTAASATW (SEQ ID NO: 15) (MW: 1284, PI: 5.52) peptide is a SIMP1 mutated version peptide wherein K138 residue has been replaced by Alanine and it displays no activity on both mutant categories.

SIMP1M:FQQSSTARSATW (SEQ ID NO:16) (MW: 1369, PI: 9.75) peptide is a mutated version of SIMP1 peptide wherein K138 residue has been replaced by Arginine. As above said, this SIMP1 peptide mutated version displays the same, although increased, functions and has no activity on tumour cell lines containing different mutation types.

Therefore it is a specific object of the present invention a peptide comprising or consisting of FQQSSTARSATW (SEQ ID NO: 16) sequence to be used in medical field. The invention further concerns nucleotide sequence encoding for above said peptide and an expression vector comprising said nucleotide sequence.

A pharmaceutical composition comprising or consisting of peptide or vector as above defined as an active principle in combination with one or more pharmaceutically acceptable excipients and/or adjuvants is a further object of the invention. The pharmaceutical composition can further comprise one or more antitumor active principles as for example those selected from the group consisting of chemotherapeutic agents like cisplatin, adriamycin, 5-fluorouracil, and anti-proliferative agents like vitamin D.

The invention further concerns the use of the peptide or vector or composition as above defined for the preparation of a medicament for the treatment of tumours. Particularly, tumours containing at cellular level m-p53 His-273/p73 protein complex can be treated. p53 mutation screening is a procedure known to those skilled in the art. 90% of mutations detected in various neoplasias are localized in 5,6,7,8 exons within four well conserved gene regions. DNA is extracted from frozen cell or tissue samples, controlled and PCR amplified. Resulting products, are sequenced using a automated sequencer (ABI3730 sequencer) (Andersen et al., Diagn. Mol. Pathol.4.203- 211. 1995). At the end an electrophoretic pattern, where a point mutation can be easy detected as it results in a migration pattern different than the control, will be obtained.

The tumours to be treated can be, for example, those selected from the group consisting of breast, lung and colon and rectum tumours..

A combination of the peptide or vector as above defined with one or more antitumor active principles to be used simultaneously, separately or sequentially for the tumour therapy is a further object of the present invention.

According to a further embodiment the present invention concerns the use of the peptide with FQQSSTAKSATW (SEQ ID NO: 1) sequence for the preparation of a medicament to be used for the treatment of the tumours containing at cellular level m-p53 His-273/p73 protein complex.

The present invention now will be described by illustrative, but not limitative, way according to preferred embodiments thereof, with particular reference to the enclosed drawings wherein:

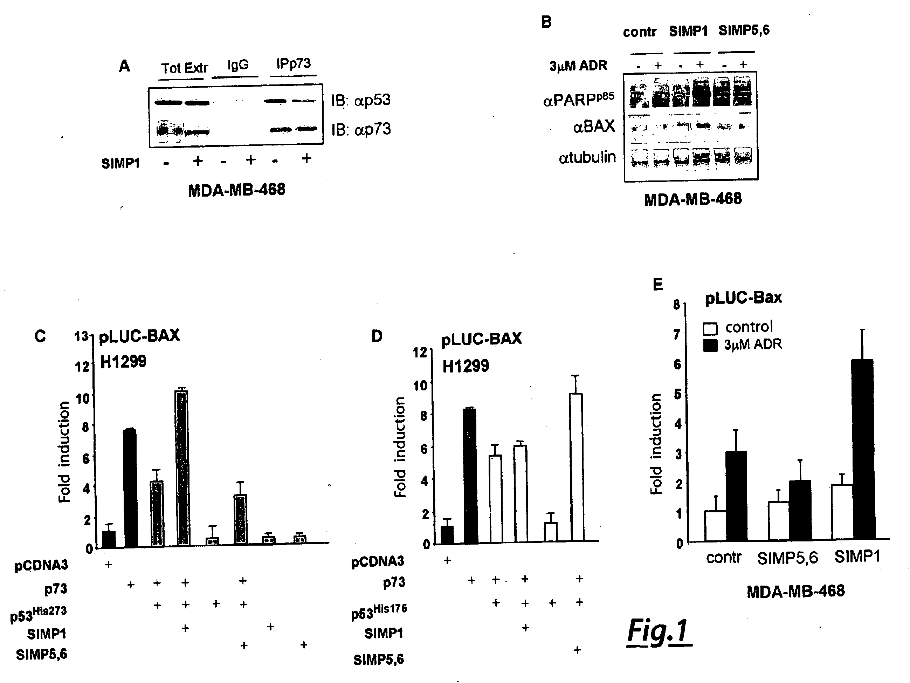

Figure 1 shows that SIMPI peptide disrupts mutated p53/p73 protein complex and increases the apoptotic response in mutant p53 His273 expressing cells. (A) MDA-MB-468 (5x10⁵) cells have been transfected using a lipofectamine containing mixture, βgal expression vector, which normalizes transfection efficiency, and SIMPI peptide (20 µM). Total cellular lysates (1 mg), have been immunoprecipitated with antibodies directed against p73 and immunoglobulins (IgG). Immunoprecipitates and an aliquot (50 µg) of total lysate after 12 % acrylamide gel electrophoretic run have been transferred on nitrocellulose membrane and labelled with antibodies shown in the figure. (B) MDA-MB-468 cells, have been transfected using SIMP1 and SIMP5,6. peptides. Successively, cells have been washed and stimulated with 3 µM Adriamycin. Total obtained lysates (30 µg) have been assayed for the expression of PARP and BAX apoptotic proteins. Lysate has been normalized by assaying γ-tubulin expression. (C-D). Transactivation tests have been carried out by transient transfection of H1299 cells using a mixture containing lipofectamine, SIMP1, SIMP5,6 indicated plasmids (20 µM each) together with pLUC-BAX reporter vector. To each reaction mixture an equal amount of β-galactosidase expression vector was added, in order to normalize the transfection efficiency.

(E) SIMP1 increases the Adriamycin sensitivity in His-273 p53 mutation containing cells. MDA-MB-468 cells have been transfected using a mixture containing lipofectamine, pLUC-BAX plasmid, β-galactosidase expression vector, SIMP1 and SIMP control peptides. The cells, 24 hours post- transfection, have been treated with 3µM Adriamycin.

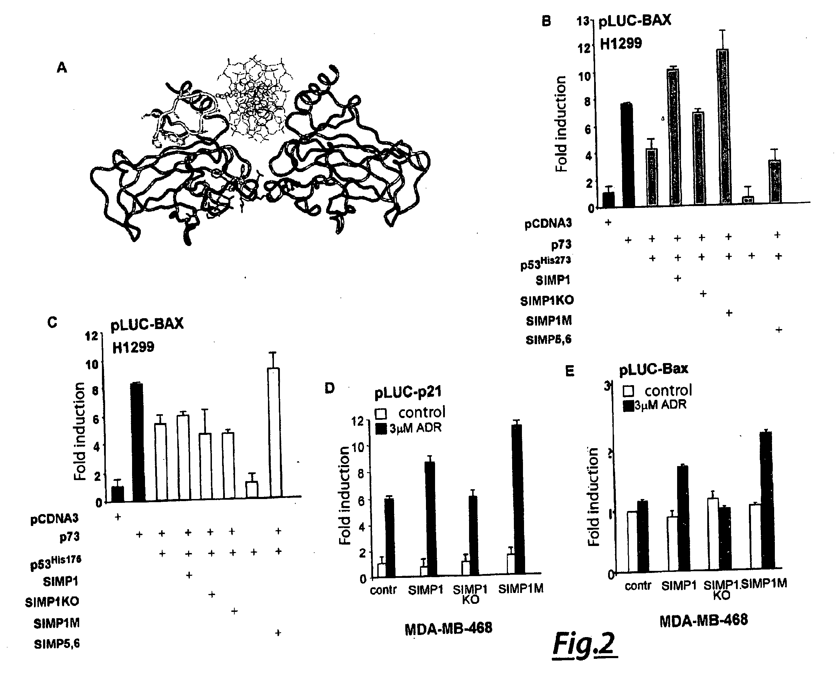

Figure 2 shows that SIMP1 and SIMP1M peptides modify mutated p53/p73 protein complex restoring p73 oncosuppressive functions.
(A) Details concerning SIMP1 mapping on DNA bound p73/wtp53 dimer model have been described in the text.
(B-C) Transactivation tests were carried out by H1299 cell transient transfection using a mixture containing lipofectamine, indicated plasmids and SIMP1, SIMP1KO, SIMP1M, SIMP5,6 peptides (20 µM each) together with pLUC-BAX reporter vector. To each reaction mixture an equal amount of β-galactosidase expression vector was added, in order to normalize the transfection efficiency..
(D-E) SIMP1 and SIMP1M but not SIMP1KO increase Adriamycin chemosensitivty in Hi-s273 p53 mutation containing cells. MDA-MB-468 cells have been transfected using a mixture containing lipofectamine, pLUC-BAX plasmid, pLUC-p21, β-galactosidase expression vector, SIMP1 peptides and SIMP1M and SIMP1KO derived mutant thereof. The cells, 24 hours post-transfection, have been treated with 3 µM Adriamicyn and then processed.

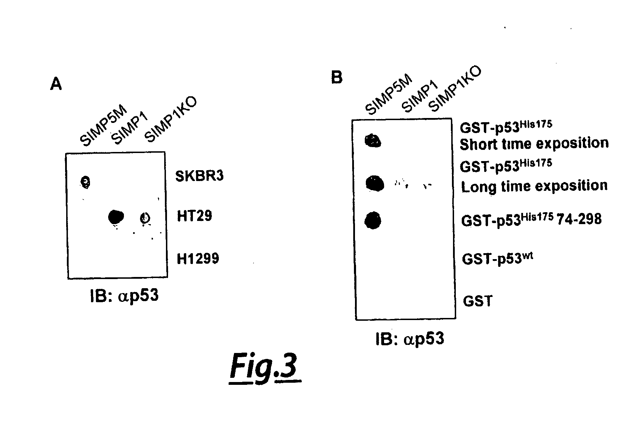

Figure 3 shows that SIMP1 during in "vitro" assays binds directly the mutp53 His-273 protein.(A) Total extracts deriving from SKBR3, (His-175) HT29, (His-273) and H1299 (p53 free) tumour cells have been incubated (1 mg) on SIMP1 and SIMP1KO peptide labelled nitrocellulose membrane and Dot Blot analysed. In (B) the membranes have been incubated with 20 µg of wild type WT GST-p53 purified protein or GST-p53 His-175,GST-p53-His-175, 74-298 or GST alone. In both experiments dots have been detected by labelling the membranes with anti-p53 (DO1) antibody

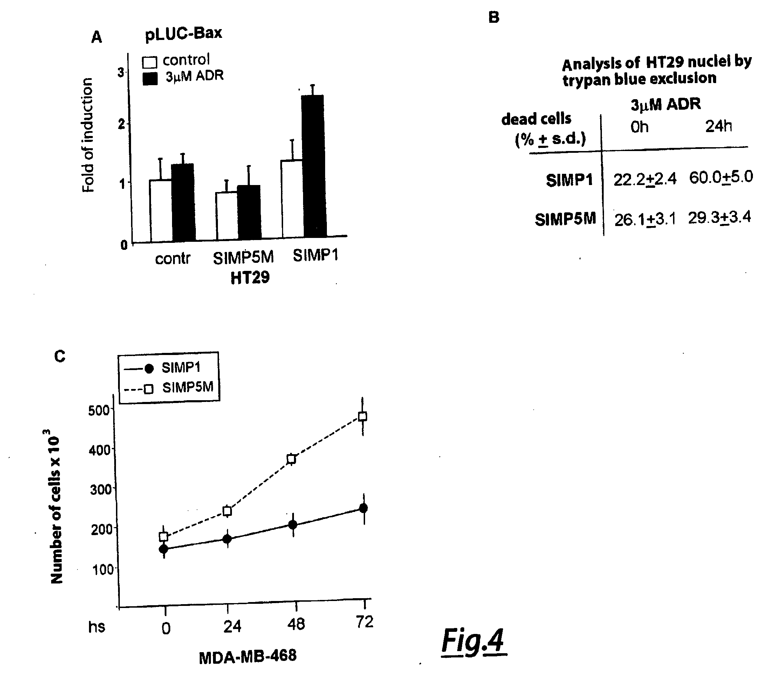

Figure 4 shows the "in vitro" SIMP1antitumor activity.
(A) In order to verify the SIMPI peptide specificity, independently from the cellular context, we tested SIMP1 peptides in some Adriamicyn treated colon carcinoma HT29 (His-273).cell lines. The cells have been transfected using a mixture containing lipofectamine, pLUC-BAX plasmid, β-galactosidase expression vector, SIMP1 and SIMP5M control peptides. 24 hours post-transfection, have been treated with 3µM Adriamicyn. (B-C). Viability and inhibition assays have been carried out after transfection using SIMP1 and SIMP5M peptides in HT29 (colon cancer) and MDA-MB-468 (breast cancer) cell lines

Figure 5 shows that "in vivo" tumour growth is decreased by SIMP1. "

In order to assay SIMP1 in vivo antitumor activity nu/nu SCID mice were administered subcutaneously with SIMP1, SIMP1KO and SIMP5M transfected HT29 cells (5.0x10⁵ cells/mouse). Tumour growth has been monitored over 5 weeks.

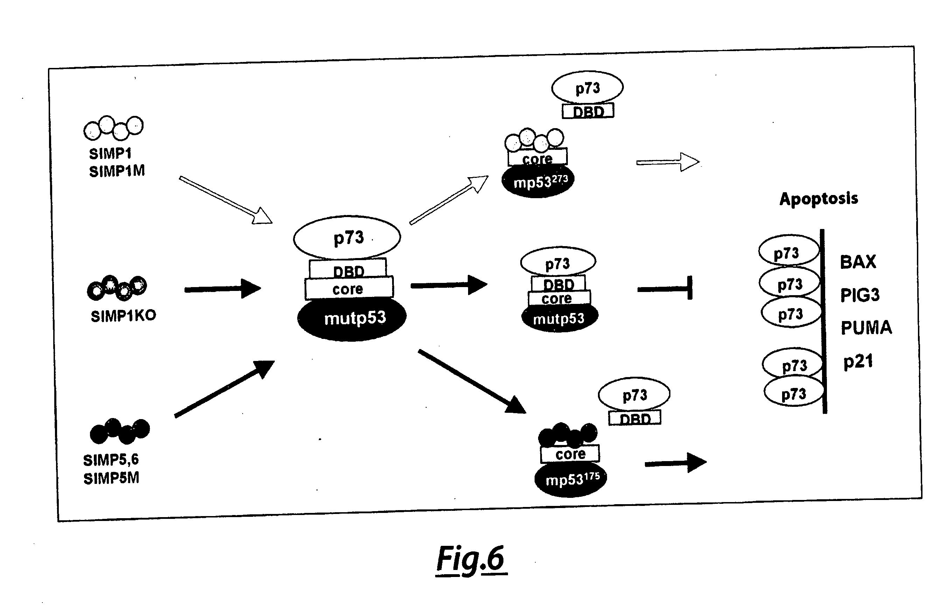

Figure 6 that SIMP peptides and by-products thereof displace protein complexes involving mutated protein p53 and p73. In particular, SIMP5,6 and SIMP5M peptides are specific for His-175 mutation containing tumours; while SIMP1 and SIMP1M peptides display specificity for His-273 mutation containing tumours.

### EXAMPLE 1: Synthesis of SIMP1 and SIMP1M peptides and study about pharmacological activity thereof

### MATERIALS AND METHODS

### Cell cultures and treatments

H1299, SKBR3, HT29, MDA-MB-468 (ATCC, USA) tumour cell lines have been cultured at 37 °C in 5% humidified CO₂ atmosphere in DMEM modified Eagle's medium (DMEM, Hyclone, Logan, LT) added with 2 mM L-glutamine, 100 IU/ml, penicillin-streptomycin (Gibco, Gaithersburg, MD) and 10% foetal bovine serum (FCS, Hyclone) as previously reported (Strano et al., 2000; DiAgostino et al., 2006). Cell lines have been treated over 24 hours with various Adriamicyn concentrations (ADR; 3,5 µM Sigma).

Cell viability has been tested using Trypan Blue exclusion assays.

### p53 mutation screening

DNA has been extracted from cell o tissue frozen samples, controlled and PCR amplified. Resulting products have been sequenced using an automated sequencer (ABI3730 sequencer) (Andersen et al., Diagn. Mol. Pathol.4. 203-211. 1995).. At the end an electrophoretic pattern, where a point mutation can be easy detected as it results in a migration pattern different than the control, will be obtained.

### Plasmids and Transfection

For transfection experiments the following plasmids have been used: pcDNA3, pcDNA3-p53His-175, pcDNA3-p53His-273, pcDNA3-p73 and pLuc-Bax promoter, pLuc-p21 promoter (from Prof. Kaelin B., Harvard Medical School and Oren M. Weizmann Institute, Rehovot laboratories). H1299 cell line has been transiently transfected according to calcium phosphate procedure in the presence of BES (Sigma). Precipitates have been left in culture medium over 12 hours after medium substitution with RPMI-160 added with 10% FCS. Finally, the cells have been lysed after 36 or 48 hours. (Strano et al., Journal Biological Chemistry 275:29503-508 2000).

### Transactivation tests

SKBR3, HT-29, MDA-MB-468 cell lines have been transfected with Lipofectamine 2000 according to the supplier instructions (InVitrogen).

Cells (1.5x10⁵) have been transiently transfected using SIMP peptides, expression plasmids and reporter constructs as internal controls for transfection efficiency. After the transfection, SKBR3, HT-29 and MDA-MB-468 have been incubated with 3 µM ADR over 24 hours. Cells have been washed with cold PBS and re-suspended in lysis buffer (Promega) and incubated for 10 min at room temp.. Insoluble material has been centrifuged and luciferase activity has been determined using a commercial kit with a luminometer (Turner).

### Peptides

Peptides have been designed by analyzing the sequence of DNA binding domain of human p73 (131-310 aa). The peptides after purification and characterization using mass spectrometry analysis, have been transfected with Lipofectamine 2000 according to the supplier instructions using 10 to 40 µM concentration for each sample.

### Synthesis of Peptides

Solid phase peptide synthesis has been carried out on Pioneer TMP Peptide Synthesis System automated peptide synthesizer using Fmoc technique for chain assembling. According to this method, Nα amino group is temporarily protect by 9-fluorenylmetoxy carbonyl (Fmoc) group. The first chain assembling step is the deprotection or removal of Fmoc protecting group by means of piperidine. Fmoc group is an unstable base that can be readily removed using a secondary amine, as well as with 20% piperidine in N,N-dimethyl formamide. Peptide chain is assembled by reaction of unprotected N-terminal group of solid support bound amino acid with C-carboxyl group. The peptides have been obtained by chemical synthesis from the C-terminal end towards N-terminal end with alpha-carboxyl group of amino acid bound to solid support consisting of 1% divinylbenzene polystyrene or polyethylene resin or on polystyrene support. Before the coupling, the amino acid carboxyl group has been activated using HOBt/DCC or HATU/DIEA and PyBop/DIEA methods.

The deprotection and detachment are crucial steps for peptide synthesis. There are several chemical activating agents and the choice is dependent on various factors: in this case 1-hydroxybenzotriazole (HOBt) has been used. **Not-blocked process of N-terminal group, carboxyl group activation and bond formation with successive amino acids have been repeated up to the obtainment of the interest peptide.** At the completion of the synthesis the peptide has been detached from the support and de-protected using a TFA/anisole/mercaptoethanol mixture. Obtained peptides have been isolated from TFA solution by cold ether precipitation or chloroform extraction. Successively, HPLC purified peptides have been eluted from sephasil C8 column using an acetonitrile water linear gradient at pH 3. A aliquot of resulting sample has been hydrolyzed at 108°C for at least 24 hours using 6 N HCl and analyzed in order to test the exact amino acid composition using HPLC with fluorescence detector.

### Preparation of cell extracts and western blot analysis

The cell extracts have been processed in a lysis buffer containing 50 mm Tris HCl pH 8, 100 mM NaCl, 10% glycerol, 1% Triton X-100, 1 mM EDTA, 100 mM NaF, MgO₂, 1 mM PMSF, protease and phosphatase inhibitors. Then extracts have been sonicated and centrifuged at 14.000 rpm for 10 min for the debris removal.

50 µg of solubilised proteins have been separated by electrophoresis on 10 or 15% SDS-polyacrylamide gel. Western blot analysis has been carried out using anti-p53 primary antibodies (DO1 Calbiochem), rabbit anti-PARP, rabbit polyclonal anti-Bax, anti-p21 and anti-tubulin monoclonal antibodies (Calbiochem). Goat anti-mouse and goat anti-rabbit horse peroxidase conjugated secondary antibodies (Amersham) have been used for the expression analyses. Immunolabelled lanes have been detected using chemiluminescence analysis (Pierce).

### Immunoprecipitation assays

Purified extracts have been incubated with Prot.A/G Sepharose (Pierce) in lysis buffer containing 0.05% BSA and 1 µg of p73 rabbit antibody (Saint Cruz) or with pre-immune serum after incubation over 2 hours at 4°C. Successively, immunocomplexes have been re-suspended in lysis buffer and eluted in 50 µl of SDS sample buffer for Western Blot analysis.

### Dot immunobinding tests

Nitrocellulose membranes have been cut, washed with water and blotted on the paper filters. The peptides at 250 mM concentration have been transferred on membrane. Labelled membrane has been washed for 10 min. with TBST buffer (10 mM Tris-HCl pH 7,4, 150 mM NaCl, 0,05% (v/v) Tween-20) and then blocked for 45 min with TBST/5% milk. Then the membranes have been incubated for 6 hours at 4°C in shaker with 1 mg of total protein extract or 20 µg of GST fusion purified proteins. Successively the membranes have been washed with TBST buffer and decorated with anti-p53 antibody (DO1) as a primary antibody. As secondary antibody horse peroxidase conjugated goat antibody has been used. Immunolabelled lanes have been detected using chemiluminescence analysis (Pierce).

### In vivo tumorigenicity tests

Xenograft assays have been carried out by administration to naked mice subcutaneous injections containing 5x10⁵ cells/mouse of SIMP1, SIMP1KO and SIMP5M peptide transfected colon and rectum tumour human cell line (nu/nu CD1 Charles River mice). The tumour growth together with animal body weight is monitored for 5 weeks in order to estimate the chronic toxicity

All the animal involving procedures and care thereof have been carried out according to actual institutional guidelines.

### RESULTS

### SIMP1 and SIMP1M peptides disrupt selectively m-p53 /p73 complex

In order to evaluate the activity of SIMPI peptide and SIMP1KO and SIMP1M derivatives the ability in disrupting the oncogenic m-p53His-273/p73 complex has been analysed. Coprecipitation tests showed that p73 bound m-p53 amount was largely reduced (Fig.1A) in MDA-MB-468 breast cancer cell lines containing His-273 mutation.

In order to demonstrate the alteration occurrence of m-p53/p73 protein complex, cell lysates of colon HT29 (m-p53 His273), lung H1299 (p53 null), breast SKBR3 (m-53 His175) cell lines have been incubated with nitrocellulose membrane labelled with SIMP1, SIMP1KO, SIMP1M, SIMP5M peptides (Fig. 3A). The membranes decorated with SIMP peptides also have been incubated with GST-p53 His175, GST p53His74-298, GST WTp53 fusion proteins, GST control. Detection against p53 protein showed that SIMP5M specifically reacts against his175 m-p53, no reaction was observed with SIMP1, SIMP1KO peptides (Fig 3B).

### SIMP1 restores the transcription activity of p73 gene, increasing Adriamycin induced apoptosis in m-p53 His-273 expressing cells

To this end, we have analyzed the ability in blocking the His273 p53 induced inhibition on transcription activity. Transactivation tests showed that SIMP1 transfection blocked the m-p53 His273 inhibition on p73 transcription activity. SIMP1 transfection increased p73 transcription response as an adriamycin response.

The peptides have been transfected in the cells using lipofectamine 2000 method (Invitrogen) using a concentration from 10 to 40 µM for each sample (Fig.1, Fig. 2).

SIMP1 and SIMP1M peptides increase the apoptotic response to chemotherapeutic agents in m-His 273expressing tumour cell lines.

To this end cell viability and apoptotic tests (Fig. 1B, Fig.4B-C) on MDA-MB468 breast carcinoma have been carried out.

SIMP1 and SIMP1M peptides reduce the cell proliferation in vitro and the tumour growth in vivo.

We observed a reduction of cell proliferation in MDA-MB468 breast and HT29 colon carcinoma cell lines.

Experiments carried out on naked mouse subjected to transplant with SIMP1, SIMP1M peptide treated cell lines (translated) showed a meaningful reduction of the growth (tumour size). On the contrary, experiments carried out on naked mouse inoculated with SIMP1KO or SIMP5M peptide transfected cells showed a tumour growth similar to non treated control (Fig. 4). Further the translation of SIMP1 in human HT29 tumour cell lines made the same more sensitive to the treatment with Adriamycin.

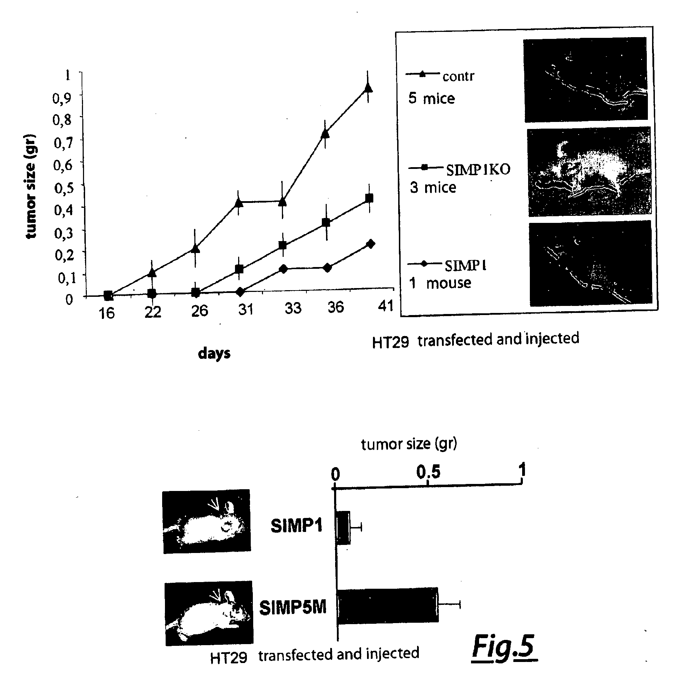

The experiments have been carried out by administration to naked mouse (nu/nu CD1 Charles River mice) subcutaneous injections containing 5 x 10⁵ cells/mouse. The tumour growth has been monitored together with animal body weight for 5 weeks in order to estimate possible not occurred chronic toxic effects (Fig.5).

## Claims

1. Peptide comprising or consisting of FQQSSTARSATW (SEQ ID NO: 16) sequence for use in the medical field.

2. Nucleotide sequence encoding for the peptide of claim 1.

3. Expression vector comprising the nucleotide sequence of claim 2.

4. Pharmaceutical composition comprising or consisting of the peptide as defined in claim 1 or vector as defined in claim 3 as an active principle in combination with or more pharmaceutical acceptable excipients and/or adjuvants.

5. Pharmaceutical composition according to claim 4, further comprising one or more chemotherapeutic agents.

6. Pharmaceutical composition according to claim 5 wherein the chemotherapeutic agents are selected from the group consisting of cisplatin, adriamycin, 5-fluorouracil or anti-proliferative agents like vitamin D.

7. Use of peptide as defined in claim 1 or vector as defined in claim 3 or composition as defined in anyone of claims 4-6 for the preparation of a medicament for the treatment of tumours.

8. Use according to claim 7, wherein the tumours display at cellular level m-p53 His273/p73 protein complex.

9. Use according to anyone of claims 7-8, wherein the tumours are selected form the group consisting of breast, lung and colon and rectum tumours.

10. Combination of the peptide as defined in claim 1 or vector as defined in claim 3 and one or more chemotherapeutic agents for the simultaneous, separated or sequential use in the treatment of tumours.

11. Use of the peptide having FQQSSTAKSATW (SEQ ID NO: 1) sequence for the preparation of a medicament for the treatment of the tumours displaying at cellular level m-p53 His273/p73 protein complex.

## Patentansprüche

1. Peptid, umfassend oder bestehend aus der Sequenz FQQS-STARSATW (SEQ ID Nr. 16), zur Verwendung auf dem Gebiet der Medizin.

2. Nukleotidsequenz, kodierend für das Peptid nach Anspruch 1.

3. Expressionsvektor, umfassend die Nukleotidsequenz von Anspruch 2.

4. Pharmazeutische Zusammensetzung, welche das Peptid wie in Anspruch 1 definiert oder den Vektor wie in Anspruch 3 definiert umfasst oder daraus besteht, als Wirkstoff in Kombination mit einem oder mehreren pharmazeutisch annehmbaren Excipienten und/oder Adjuvans/Adjuvantien.

5. Pharmazeutische Zusammensetzung nach Anspruch 4, welche ferner ein chemotherapeutisches Agens oder mehrere chemotherapeutische Agenzien umfasst.

6. Pharmazeutische Zusammensetzung nach Anspruch 5, wobei die chemotherapeutischen Agenzien aus der Gruppe ausgewählt sind, die aus Cisplatin, Adriamycin, 5-Fluoruracil und Antiproliferationsmitteln wie Vitamin D besteht.

7. Verwendung eines Peptids wie in Anspruch 1 definiert oder eines Vektors wie in Anspruch 3 definiert oder einer Zusammensetzung wie in irgendeinem der Ansprüche 4 bis 6 definiert zur Herstellung eines Medikaments für die Behandlung von Tumoren.

8. Verwendung nach Anspruch 7, wobei die Tumore auf der zellulären Ebene den Proteinkomplex m-p53 His273/p73 präsentieren.

9. Verwendung nach irgendeinem der Ansprüche 7 bis 8, wobei die Tumore aus der Gruppe ausgewählt sind, die aus Brust-, Lungen-, Dickdarm- und Rektaltumoren besteht.

10. Kombination des Peptids wie in Anspruch 1 definiert oder des Vektors wie in Anspruch 3 definiert und eines chemotherapeutischen Agens oder mehrerer chemotherapeutischer Agenzien für die gleichzeitige, separate oder sequenzielle Verwendung bei der Behandlung von Tumoren.

11. Verwendung des Peptids mit der Sequenz FQQSSTAKSATW (SEQ ID Nr. 1) zur Herstellung eines Medikaments zur Behandlung der Tumore, welche auf zellulärer Ebene den Proteinkomplex m-p53 His273/p73 präsentieren.

## Revendications

1. Peptide comprenant ou constitué de la séquence FQQSSTARSATW (SEQ ID N°: 16) à utiliser dans le domaine médical.

2. Séquence nucléotidique codant pour le peptide de la revendication 1.

3. Vecteur d'expression comprenant la séquence nucléotidique de la revendication 2.

4. Composition pharmaceutique comprenant le peptide tel que défini dans la revendication 1 ou constituée d'un tel peptide, ou le vecteur tel que défini dans la revendication 3 en tant que principe actif en combinaison avec un ou plusieurs excipients et/ou adjuvants pharmaceutiques acceptables.

5. Composition pharmaceutique selon la revendication 4, comprenant en outre un ou plusieurs agents chimiothérapeutiques.

6. Composition pharmaceutique selon la revendication 5, dans laquelle les agents chimiothérapeutiques sont choisis dans le groupe constitué par le cisplatine, l'adriamycine, le 5-fluorouracile et les agents antiprolifératifs comme la vitamine D.

7. Utilisation d'un peptide tel que défini dans la revendication 1 ou d'un vecteur tel que défini dans la revendication 3 ou d'une composition telle que définie dans l'une quelconque des revendications 4-6 pour la préparation d'un médicament destiné au traitement des tumeurs.

8. Utilisation selon la revendication 7, où les tumeurs présentent au niveau cellulaire un complexe protéique m-p53 His273/p73.

9. Utilisation selon l'une quelconque des revendications 7-8, où les tumeurs sont choisies dans le groupe constitué par les tumeurs du sein, du poumon et les tumeurs du côlon et du rectum.

10. Combinaison du peptide tel que défini dans la revendication 1 ou du vecteur tel que défini dans la revendication 3 et d'un ou de plusieurs agents chimiothérapeutiques pour l'utilisation simultanée, séparée ou séquentielle dans le traitement des tumeurs.

11. Utilisation du peptide ayant la séquence FQQSSTAKSATW (SEQ ID N°: 1) pour la préparation d'un médicament destiné au traitement des tumeurs présentant au niveau cellulaire un complexe protéique m-p53 His273/p73.
